# DEMANDE DE BREVET EUROPEEN

(11) **EP 0 665 291 A1**
(43) Date de publication de la demande: **02.08.1995**
(21) Numéro de dépôt: 95200129.5
(22) Date de dépôt: 19.01.1995
(51) Int. Cl.: C12N 15/80, C12N 15/53, C12N 15/67, C12N 1/15, C12N 9/04, C12N 9/34

(54) **Système hôte-vecteur permettant d'exprimer la glucose oxidase**

(30) Priorité: 28.01.1994 BE 9400102; 17.06.1994 BE 9400586; 09.01.1995 BE 9500014
(71) Demandeur: SOLVAY (Société Anonyme), B-1050 Bruxelles (BE)
(72) Inventeur: Carrez, Dirk, B-1800 Strombeek-Bever (BE); Roos, Joel, B-7911 Frasnes-Les-Auraing (BE)
(74) Mandataire: Lechien, Monique

(57) **Abrégé**

L'invention concerne un système d'expression, utilisable pour la production extracellulaire de glucose oxydase, qui comprend la séquence d'un promoteur, la séquence du signal de sécrétion de la glucose oxydase, la séquence mature de la glucose oxydase, et la séquence d'un terminateur. En particulier les sequences de la glucose oxydase proviennent d'une souche d'Aspergillus foetidus et les séquences du promoteur et du terminateur sont celles de la glucoamylase d'Aspergillus foetidus.

L'invention concerne également un vecteur d'intégration contenant le système d'expression et une cellule transformée par ce vecteur.

LISTE DE SEQUENCES

(1) INFORMATIONS GENERALES :

(i) DEPOSANT :
(A) NOM : SOLVAY (Société Anonyme)
(B) RUE : rue du Prince Albert, 33
(C) VILLE : Bruxelles
(E) PAYS : Belgique
(F) CODE POSTAL : 1050
(G) TELEPHONE : (02) 509.61.11

(ii) TITRE DE L'INVENTION : Système d'expression, vecteur
d'intégration et cellule transformée par ce vecteur d'intégration

(iii) NOMBRE DE SEQUENCES : 8

(iv) FORME LISIBLE PAR ORDINATEUR :
(A) TYPE DE SUPPORT : Floppy disk
(B) ORDINATEUR : IBM PC compatible
(C) SYSTEME D'EXPLOITATION : PC-DOS:MS-DOS

(2) INFORMATION POUR LA SEQ ID NO:1:

(i) CARACTERISTIQUES DE LA SEQUENCE :
(A) LONGUEUR : 63 paires de bases
(B) TYPE : acide nucléique
(C) NOMBRE DE BRINS : simple
(D) CONFIGURATION : linéaire

(ii) TYPE DE MOLECULE : acide nucléique (oligonucléotide
synthétique)

(xi) DESCRIPTION DE LA SEQUENCE: SEQ ID NO:1:

TGATGATCAG GATCCGGCGG CCGCACCTCA GCAATGCAGA CTCTCCTTGT GAGCTCGCTT 60

GTG 63

(2) INFORMATION POUR LA SEQ ID NO:2:

(i) CARACTERISTIQUES DE LA SEQUENCE :
(A) LONGUEUR : 51 paires de bases
(B) TYPE : acide nucléique
(C) NOMBRE DE BRINS : simple
(D) CONFIGURATION : linéaire

(ii) TYPE DE MOLECULE : acide nucléique (oligonucléotide
synthétique)

(xi) DESCRIPTION DE LA SEQUENCE: SEQ ID NO:2:

TCGATGAAGC TTCACTCACT GCATGGAAGC ATAATCTTCC AAGATAGCAT C 51

(2) INFORMATION POUR LA SEQ ID NO:3:

(i) CARACTERISTIQUES DE LA SEQUENCE :
(A) LONGUEUR : 51 paires de bases
(B) TYPE : acide nucléique
(C) NOMBRE DE BRINS : simple
(D) CONFIGURATION : linéaire

(ii) TYPE DE MOLECULE : acide nucléique (oligonucléotide
synthétique)

(xi) DESCRIPTION DE LA SEQUENCE: SEQ ID NO:3:

GATGCTATCT TGGAAGATTA TGCTTCCATG CAGTGAGTGA AGCTTCATCG A 51

(2) INFORMATION POUR LA SEQ ID NO:4:

(i) CARACTERISTIQUES DE LA SEQUENCE :
(A) LONGUEUR : 25 paires de bases
(B) TYPE : acide nucléique
(C) NOMBRE DE BRINS : simple
(D) CONFIGURATION : linéaire

(ii) TYPE DE MOLECULE : acide nucléique (oligonucléotide
synthétique)

(xi) DESCRIPTION DE LA SEQUENCE: SEQ ID NO:4:

GATTCATGGT TGAGCAACGA AGCGA 25

(2) INFORMATION POUR LA SEQ ID NO:5:

(i) CARACTERISTIQUES DE LA SEQUENCE :
(A) LONGUEUR : 49 paires de bases
(B) TYPE : acide nucléique
(C) NOMBRE DE BRINS : simple
(D) CONFIGURATION : linéaire

(ii) TYPE DE MOLECULE : acide nucléique (oligonucléotide
synthétique)

(xi) DESCRIPTION DE LA SEQUENCE: SEQ ID NO:5:

GCCTGAGCTT CATCCCCAGC GCGGCCGCAT CATTACACCT CAGCAATGT 49

(2) INFORMATION POUR LA SEQ ID NO:6:

(i) CARACTERISTIQUES DE LA SEQUENCE :

(A) LONGUEUR : 50 paires de bases
(B) TYPE : acide nucléique
(C) NOMBRE DE BRINS : simple
(D) CONFIGURATION : linéaire

(ii) TYPE DE MOLECULE : acide nucléique (oligonucléotide synthétique)

(xi) DESCRIPTION DE LA SEQUENCE: SEQ ID NO:6:

TGACTGACAC CTGGCGGTGA AAGCTTCAAT CAATCCATTT CGCTATAGTT 50

(2) INFORMATION POUR LA SEQ ID NO:7:

(i) CARACTERISTIQUES DE LA SEQUENCE :
(A) LONGUEUR : 2045 paires de bases
(B) TYPE : acide nucléique
(C) NOMBRE DE BRINS : simple
(D) CONFIGURATION : linéaire

(ii) TYPE DE MOLECULE : ADN génomique

(xi) DESCRIPTION DE LA SEQUENCE: SEQ ID NO:7:

GAATTCCCGA CATCGGTCAT TGGCCTCCTC GCTGATCTCC CTTTGGTACA GTCGGCTACC 60

AATGCTCTCG AGGATTGCCT GAACATTGAC ATTCGGCGTC CGGCCGGGAC CACCGCGGAC 120

TCGAAGCTGC CTGTGCTGGT CTGGATCTTT GGCGGAGGCT TTGAACTTGG TTCAAAGGCG 180

ATGTATGATG GTACAACGAT GGTATCATCG TCGATAGACA AGAACATGCC TATCGTGTTT 240

GTAGCAATGA ATTATCGCGT GGGAGGTTTC GGGTTCTTGC CCGGAAAGGA GATCCTGGAG 300

GACGGGTCCG CGAACCTAGG GCTCCTGGAC CAACGCCTTG CCCTGCAGTG GGTTGCCGAC 360

AACATCGAGG CCTTTGGTGG AGACCCGGAC AAGGTGACGA TTTGGGGAGA ATCAGCAGGA 420

GCCATTCGTT TGACTAGATG ACTTGTACGA CGGAAACATC ACTTACAAGG ATAAGCCCTT 480

GTTCCGGGGG GCCATCATGG ACTCCGGTAG TGTTGTTCCC GCAGACCCCG TCGATGGGGT 540

CAAGGGACAG CAAGTATATG ATGCGGTAGT GGAATCTGCA GGCTGTTCCT CTTCTAACGA 600

CACCCTAGCT TGTCTGCGTG AACTAGACTA CACCGACTTC CTCAATGCGG CAAACTCCGT 660

GCCAGGCATT TTAAGCTACC ATTCTGTGGC GTTATCATAT GTGCCTCGAC CGGACGGGAC 720

GGCGTTGTCG GCATCACCGG ACGTTTTGGG CAAAGCAGGG AAATATGCTC GGGTCCCGTT 780

CATCGTGGGC GACCAAGAGG ATGAGGGGAC CTTATTCGCC TTGTTTCAGT CCAACATTAC 840

GACGATCGAC GAGGTGGTCG ACTACCTGGC CTCATACTTC TTCTATGACG CTAGCCGAGA 900

GCAGCTTGAA GAACTAGTGG CCCTGTACCC AGACACCACC ACGTACGGGT CTCCGTTCAG 960

ACAGCGCGGC CAACAACTGG TATCCGCAAT TTAAGCGATT GGCCGCCATT CTCGGCGACT 1020

TGGTCTTCAC CATTACCGGC GGGCATTCCT CTCGTATGCA GAGGAAATCT CCCCTGATCT 1080

TCCGAACTGG TCGTACCTGG CGACCTATGA CTATGGCACC CCAGTTCTGG GGACCTTCCA 1140

CGGAAGTGAC CTGCTGCAGG TGTTCTATGG GATCAAGCCA AACTATGCAG CTAGTTCTAG 1200

CCACACGTAC TATCTGAGCT TTGTGTATAC GCTGGATCCG AACTCCAACC GGGGGGAGTA 1260

CATTGAGTGG CCGCAGTGGA AGGAATCGCG GCAGTTGATG AATTTCGGAG CGAACGACGC 1320

CAGTCTCCTT ACGGATGATT TCCGCAACGG GACATATGAG TTCATCCTGC AGAATACCGC 1380

GGCGTTCCAC ATCTGATGCC ATTGGCGGAG GGGTCCGGAC GGTCAGGAAC TTAGCCTTAT 1440

GAGATGAATG ATGGACGTGT CTGGCCTCGG AAAAGGATAT ATGGGATCAT GATAGTACTA 1500

GCCATATTAA TGAAGGGCAT ATACCACGCG TTGGACCTGC GTTATAGCTT CCCGTTAGTT 1560

ATAGTACCAT CGTTATACCA GCCAATCAAG TCACCACGCA CGACCGGGGA CGGCGAATCC 1620

CCGGGAATTG AAAGAAATTG CATCCCAGGC CAGTGAGGCA GCGATTGGCC ACCTCTCCAA 1680

GGCACAGGGC CATTCTGCAG CGCTGGTGGA TTCATCGCAA TTTCCCCCGG CCCGGCCCGA 1740

CACCGCTATA GGCTGGTTCT CCCACACCAT CGGAGATTCG TCGCCTAATG TCTCGTCCGT 1800

TCACAAGCTG AAGAGCTTGA AGTGGCGAGA TGTCTCTGCA GGAATTCAAG CTAGATGCTA 1860

AGCGATATTG CATGGCAATA TGTGTTGATG CATGTGCTTC TTCCTTCAGC TTCCCCTCGT 1920

GCAGATGAGG TTTGGCTATA AATTGAAGTG GTTGGTCGGG GTTCCGTGAG GGGCTGAAGT 1980

GCTTCCTCCC TTTTAGACGC AACTGAGAGC CTGAGCTTCA TCCCCAGCAT CATTACACCT 2040

CAGCA 2045

(2) INFORMATION POUR LA SEQ ID NO:8:

(i) CARACTERISTIQUES DE LA SEQUENCE :
(A) LONGUEUR : 1035 paires de bases
(B) TYPE : acide nucléique
(C) NOMBRE DE BRINS : simple
(D) CONFIGURATION : linéaire

(ii) TYPE DE MOLECULE : ADN génomique

(xi) DESCRIPTION DE LA SEQUENCE: SEQ ID NO:8:

CAATCAATCC ATTTCGCTAT AGTTAAAGGA TGGGGATGAG GGCAATTGGT TATATGATCA 60

TGTATGTAGT GGGTGTGCAT AATAGTAGTG AAATGGAAGC CAGTCATGTG ATTGTAATCG 120

ACCGACGGAA TTGAGGATAT CCGGAAATAC AGACACCGTG AAAGCCATGG TCTTTCCTTC 180

GTGTAGAAGA CCAGACAGAC AGTCCCTGAT TTACCCTTGC ACAAAGCACT AGAAAATTAG 240

CATTCCATCC TTCTCTGCTT GCTCTGCTGA TATCACTGTC ATTCAATGCA TAGCCATGAG 300

CTCATCTTAG ATCCAAGCAC GTAATTCCAT AGCCGAGGTC CACAGGTGAG CAGCAACATT 360

CCCCATCATT GCTTTCCCAG GGCCTCCCAA CGACTAAATC AAGAGTATAT CTCTACCGTC 420

CAATAGATCG TCTTCGCTTC AAAATCTTTG ACAATTCCAA GAGGGTCCCC ATCCATCAAA 480

CCCAGTTCAA TAATAGCCGA GATGCATGGT GGAGTCAATT AGGCAGTATT GCTGGAATGT 540

CGGGGCCAGT TCCGGTGGTC ATTGGCCGCC TGTGATGCCA TCTGCCACTA AATCCGATCA 600

TTGATCCACC GCCCACGAGG CGCGTCTTTG CTTTTTGCGC GGCGTCCAGG TTCAACTCTC 660

TCTGCAGCTC CAGTCCAACG CTGACTGACT AGTTTACCTA CTGGTCTGAT CGGCTCCATC 720

AGAGCTATGG CGTTATCCCG TGCCGTTGCT GCGCAATCGC TATCTTGATC GCAACCTTGA 780

ACTCACTCTT GTTTTAATAG TGATCTTGGT GACGGAGTGT CGGTGAGTGA CAACCAACAT 840

CGTGCAAGGG AGATTGATAC GGAATTGTCG CTCCCATCAT GATGTTCTTG CCGGCTTTGT 900

TGGCCCTATC GTGGGATCGG ATGCCCTCGC TGTGCAGCAG CAGGTACTGC TGGATGAGGA 960

GCCATCGGTC TCTGCACGCA AACCCAACTT CCTCTTCATT CTCACGGATG ATCAGGATCT 1020

CCGGATGAAG AATTC 1035

## Description

L'invention concerne un système d'expression utilisable pour la production de glucose oxydase et, plus particulièrement, capable d'effectuer la production extracellulaire de la glucose oxydase.

L'invention concerne également un vecteur d'intégration contenant ce système d'expression et une cellule transformée par ce vecteur d'intégration.

La glucose oxydase est classée dans le système international selon la référence E.C. 1.1.3.4. ou β-D-glucose:oxygène-1-oxydoréductase. Cette enzyme catalyse la réaction d'oxydation du β-D-glucose en D-glucono-δ-lactone qui est ensuite hydrolysé en acide gluconique. Cette réaction produit du peroxyde d'hydrogène, qui est transformé en oxygène et en eau par l'action de la catalase produite par les souches d'Aspergillus sauvages.

La glucose oxydase est naturellement produite par certaines souches de champignons filamenteux et notamment par des souches d'Aspergillus. Malheureusement, la glucose oxydase produite par ces souches d'Aspergillus n'est pas sécrétée de manière efficace dans le milieu de culture de ces souches, la glucose oxydase ne traversant pas la paroi cellulaire (WITTEVEEN C.F.B. et al., Applied and Environmental Microbiology, 1992, 58 (4), pages 1190-1194). Industriellement la glucose oxydase est donc traitée comme une enzyme intracellulaire. Les cellules d'Aspergillus doivent être disloquées préalablement à la récupération et à la purification de la glucose oxydase. L'étape supplémentaire de dislocation des cellules, avant la récolte, est contraignante et difficile à réaliser industriellement, par ailleurs elle entraîne des chutes de rendement assez importantes. En effet, après la dislocation des cellules, la suspension aqueuse obtenue contient la glucose oxydase en solution ainsi que d'autres enzymes et des polypeptides, mais également beaucoup de constituants cellulaires plus ou moins solubles. Une étape de purification comportant plusieurs opérations de séparation est donc indispensable pour obtenir la glucose oxydase pure ou industriellement utilisable. C'est pourquoi une production extracellulaire de la glucose oxydase, c'est-à-dire la sécrétion de l'enzyme dans le milieu de culture, a longtemps été recherchée.

Par ailleurs, une méthode de production de glucose oxydase dans laquelle de la catalase ne serait présente qu'en quantités minimales dans le milieu, a longtemps été recherchée. Ceci a pour but d'éviter que le peroxyde d'hydrogène, formé lors de la réaction catalysée par la glucose oxydase, ne soit dégradé en oxygène et en eau par la catalase présente dans le milieu.

Dans ce contexte, on propose dans la demande de brevet WO 89/12675 de transformer une levure avec un plasmide. Ce plasmide contient la séquence du promoteur d'une déhydrogénase (ADH2-GAP) de levure, la séquence du signal de sécrétion de la glucose oxydase d'Aspergillus niger ou la séquence du signal de sécrétion de l'"α-mating factor" de Saccharomyces cerevisiae, la séquence mature de la glucose oxydase d'Aspergillus niger et le terminateur d'une déhydrogénase (GAP) de levure. La levure transformée (Saccharomyces cerevisiae) sécrète la glucose oxydase.

Dans la demande de brevet européen 0 357 127, on décrit un système d'expression utilisable pour la production de chymosine bovine. Ce système d'expression comprend la séquence du promoteur de la glucoamylase d'Aspergillus niger, la séquence du peptide signal de la glucoamylase d'Aspergillus niger, la séquence codante de la chymosine bovine et la séquence du terminateur de la glucoamylase d'Aspergillus niger. Une souche transformée d'Aspergillus niger, contenant ce système d'expression, permet d'obtenir la chymosine bovine.

A l'heure actuelle, un système d'expression permettant d'obtenir l'expression et une sécrétion efficace de la glucose oxydase par une souche transformée de champignon filamenteux contenant ce système d'expression, n'est pas connu.

La présente invention a pour premier but de fournir un système d'expression permettant d'obtenir la glucose oxydase dans le milieu de culture, où la souche transformée contenant ce système d'expression est cultivée. Ce système d'expression permet d'obtenir une production substantiellement extracellulaire de la glucose oxydase. Ce système d'expression permet de plus d'obtenir une composition enzymatique contenant de la glucose oxydase pratiquement sans catalase.

Par glucose oxydase, on entend une enzyme qui catalyse la réaction d'oxydation du β-D-glucose en D-glucono-δ-lactone qui est ensuite hydrolysé en acide gluconique. Cette enzyme est classée dans le système international selon la référence E.C. 1.1.3.4. ou β-D-glucose:oxygène-1-oxydoréductase. Cette définition inclut les enzymes naturelles et les enzymes modifiées, telles que les enzymes dont la séquence de nucléotides ou d'acides aminés a été modifiée par des techniques de génie génétique ou par des techniques de mutagénèse.

La présente invention a également pour but de fournir un vecteur d'intégration et un plasmide contenant le système d'expression décrit ci-dessus.

La présente invention a également pour but de fournir une souche transformée par le vecteur d'intégration ou le plasmide ci-dessus, de préférence une souche transformée d'Aspergillus, et plus particulièrement une souche transformée d'Aspergillus foetidus, qui exprime et sécrète dans son milieu de culture la glucose oxydase avec un haut niveau de productivité. La sécrétion de la glucose oxydase par cette souche transformée est substantiellement extracellulaire.

La présente invention a également pour but de fournir un procédé de production de glucose oxydase mettant en oeuvre une souche de champignon filamenteux qui produit beaucoup de glucose oxydase et la sécrète presque complètement de façon extracellulaire.

A cet effet, l'invention concerne un système d'expression utilisable pour la production extracellulaire de glucose oxydase, caractérisé en ce qu'il comprend au moins :
- la séquence d'un promoteur,
- la séquence du signal de sécrétion de la glucose oxydase, et
- la séquence mature de la glucose oxydase.

Habituellement, le système d'expression comprend également la séquence d'un terminateur.

De préférence, le promoteur est celui de la glucoamylase.

L'invention concerne un système d'expression utilisable pour la production de glucose oxydase, caractérisé en ce qu'il comprend au moins :
- la séquence du promoteur de la glucoamylase d'Aspergillus foetidus,
- la séquence du signal de sécrétion de la glucose oxydase d'Aspergillus foetidus,
- la séquence mature de la glucose oxydase d'Aspergillus foetidus, et
- la séquence du terminateur de la glucoamylase d'Aspergillus foetidus.

Par système d'expression, on entend une unité moléculaire qui peut être intégrée dans le génome d'un champignon filamenteux et qui est capable de fournir à ce champignon l'information génétique nécessaire à la biosynthèse de la glucose oxydase.

Par promoteur, on entend le ou les promoteur(s) de transcription. Le promoteur qui est constitué d'une séquence d'ADN montrant une forte activité de transcription, peut être précédé par des séquences d'ADN qui stimulent la transcription, telles que des séquences connues sous le nom de "enhancers" ou "Upstream Activating Sequences". Le promoteur contient des séquences de contrôle de transcription qui influent sur l'expression de l'ADN codant fusionné. La séquence du promoteur d'un gène, tel que celui de la glucoamylase, comprend les séquences qui contrôlent la transcription et qui interviennent lors de l'expression de ce gêne, tel que celui de la glucoamylase.

Habituellement, la séquence du promoteur provient d'un champignon filamenteux. Généralement, elle provient d'une souche d'Aspergillus. De préférence, elle provient d'une souche d'Aspergillus niger, d'Aspergillus foetidus, d'Aspergillus awamori ou d'Aspergillus oryzae. De manière particulièrement préférée, elle provient d'une souche d'Aspergillus niger ou d'une souche d'Aspergillus foetidus. Des souches d'Aspergillus niger et des souches d'Aspergillus foetidus, d'où peut provenir la séquence du promoteur de la glucoamylase, sont connues, telles que notamment la souche d'Aspergillus niger NRRL 3 (AGRICULTURAL RESEARCH SERVICE CULTURE COLLECTION (NRRL), 1815 North University Street, Peoria, Illinois 61604, USA), la souche d'Aspergillus niger ATCC 13496 (AMERICAN TYPE CULTURE COLLECTION, 12301 Parklawn drive, Rockville, Maryland 20852-1776, USA), la souche d'Aspergillus niger ATCC 22343, la souche d'Aspergillus niger ATCC 46890, la souche d'Aspergillus foetidus ATCC 10254, la souche d'Aspergillus foetidus ATCC 14916. De préférence, la séquence du promoteur de la glucoamylase provient d'une souche d'Aspergillus foetidus. De manière particulièrement préférée, elle provient de la souche d'Aspergillus foetidus SE4. La molécule d'ADN comprenant la séquence de nucléotides SEQ ID NO:7 code pour le promoteur de la souche d'Aspergillus foetidus SE4.

L'invention concerne également le promoteur de la glucoamylase d'Aspergillus foetidus. Plus particulièrement, l'invention concerne une molécule d'ADN comprenant la séquence de nucléotides SEQ ID NO:7 qui code pour le promoteur de la glucoamylase d'Aspergillus foetidus SE4.

Par la séquence du signal de sécrétion de la glucose oxydase, on entend une séquence d'ADN correspondant à une séquence d'acides aminés qui est naturellement liée de façon opérationnelle à la terminaison aminée de la séquence mature de la glucose oxydase. Le gêne codant pour la glucose oxydase a été cloné et séquencé, la séquence en acides aminés qui a été déduite de cette étude montre la présence d'une préséquence ayant certaines caractéristiques d'un peptide signal, mais plus complexes (WHITTINGTON H. et al., Curr. Genet., 18 (1990), pages 531-536). C'est pourquoi, dans cette demande, la partie du gène de structure, qui code pour le peptide signal supposé, est nommée "séquence du signal de sécrétion de la glucose oxydase" et la partie de gène de structure, qui code pour la glucose oxydase en tant que telle, est nommée "séquence mature de la glucose oxydase".

Habituellement, la séquence du signal de sécrétion de la glucose oxydase provient d'une souche de champignon filamenteux. Généralement, elle provient d'une souche d'Aspergillus ou d'une souche de Penicillium. De préférence, elle provient d'une souche d'Aspergillus, telle que notamment d'une souche d'Aspergillus niger, d'Aspergillus foetidus, d'Aspergillus awamori ou d'Aspergillus oryzae. De manière particulièrement préférée, elle provient d'une souche d'Aspergillus niger ou d'une souche d'Aspergillus foetidus. De bons résultats ont été obtenus avec la séquence du signal de sécrétion de la glucose oxydase de la souche d'Aspergillus niger NRRL 3 (AGRICULTURAL RESEARCH SERVICE CULTURE COLLECTION (NRRL), 1815 North University Street, Peoria, Illinois 61604, USA). Cette séquence a été publiée dans The Journal of Biological Chemistry, 1990, 265 (7), pages 3793- 3802. De bons résultats ont également été obtenus avec la séquence du signal de sécrétion de la glucose oxydase de la souche d'Aspergillus foetidus ATCC 14916 (AMERICAN TYPE CULTURE COLLECTION, 12301 Parklawn Drive, Rockville, Maryland 20852-1776, USA).

Le système d'expression selon l'invention comprend la séquence mature de la glucose oxydase. Par séquence mature de la glucose oxydase, on entend la partie de la séquence qui est traduite en protéine active, c'est-à-dire la séquence codante de la glucose oxydase qui correspond au gêne de structure de la glucose oxydase sans la séquence du signal de sécrétion. La séquence codante (gène de structure) comprend la séquence du signal de sécrétion et la séquence mature de la glucose oxydase.

Habituellement, la séquence mature de la glucose oxydase provient d'une souche de champignon filamenteux. Généralement, elle provient d'une souche d'Aspergillus ou d'une souche de Penicillium. De préférence, elle provient d'une souche d'Aspergillus, telle que notamment d'une souche d'Aspergillus niger, d'Aspergillus foetidus, d'Aspergillus awamori ou d'Aspergillus oryzae. De manière particulièrement préférée, elle provient d'une souche d'Aspergillus niger ou d'une souche d'Aspergillus foetidus. De bons résultats ont été obtenus avec la séquence mature de la glucose oxydase de la souche d'Aspergillus niger NRRL 3. Cette séquence a été publiée dans The Journal of Biological Chemistry, 1990, 265 (7), pages 3793-3802. De bons résultats ont également été obtenus avec la séquence mature de la glucose oxydase de la souche d'Aspergillus foetidus ATCC 14916 (AMERICAN TYPE CULTURE COLLECTION).

Dans une variante préférée de l'invention, la séquence du signal de sécrétion de la glucose oxydase et la séquence mature de la glucose oxydase proviennent du même champignon filamenteux. D'excellents résultats ont été obtenus avec un système d'expression, selon l'invention, comprenant la séquence du signal de sécrétion de la glucose oxydase de la souche d'Aspergillus foetidus ATCC 14916 et la séquence mature de la glucose oxydase de la souche d'Aspergillus foetidus ATCC 14916. D'excellents résultats ont également été obtenus avec un système d'expression, selon l'invention, comprenant la séquence du signal de sécrétion de la glucose oxydase de la souche d'Aspergillus niger NRRL 3 et la séquence mature de la glucose oxydase de la souche d'Aspergillus niger NRRL 3.

Par terminateur, on entend le ou les terminateur(s) de transcription. La séquence du terminateur d'un gène est une séquence d'ADN qui agit pour terminer la transcription de ce gène. La séquence du terminateur contient également le signal de polyadénylation qui a pour but de stabiliser le mARN.

Habituellement, la séquence du terminateur provient d'un champignon filamenteux. Dans cette demande, n'importe quel terminateur fonctionnel dans un champignon filamenteux peut convenir. Des terminateurs sont connus de l'homme du métier. Comme exemples de terminateur d'origine fongique connu, on peut citer le terminateur trpC, celui d'une glucoamylase, celui d'une amylase, celui d'une alpha-amylase, celui d'une protéase aspartique, celui d'une phosphatase acide, celui d'une lipase, celui d'une cellulase, celui d'une enzyme glycolytique, celui d'une glucanase, celui d'une hydrolase, celui d'une déhydrogénase, et en particulier, le terminateur trpC d'Aspergillus nidulans, le terminateur de la glucoamylase d'Aspergillus niger, celui de l'alpha-amylase d'Aspergillus niger, celui de la protéase aspartique de Mucor miehei, celui de la phosphatase acide d'Aspergillus niger, celui d'une alcool-déhydrogénase d'Aspergillus nidulans. Généralement, la séquence du terminateur provient d'une souche d'Aspergillus. De préférence, elle provient d'une souche d'Aspergillus niger, d'Aspergillus foetidus, d'Aspergillus awamori ou d'Aspergillus oryzae. De manière particulièrement préférée, elle provient d'une souche d'Aspergillus niger ou d'une souche d'Aspergillus foetidus. Dans une variante préférée, la séquence du terminateur est la séquence du terminateur de la glucoamylase. De préférence, la séquence du terminateur de la glucoamylase provient d'une souche d'Aspergillus foetidus. De manière particulièrement préférée, elle provient de la souche d'Aspergillus foetidus SE4. La molécule d'ADN comprenant la séquence de nucléotides SEQ ID NO:8 code pour le terminateur de la souche d'Aspergillus foetidus SE4.

L'invention concerne également le terminateur de la glucoamylase d'Aspergillus foetidus. Plus particulièrement, l'invention concerne une molécule d'ADN comprenant la séquence de nucléotides SEQ ID NO:8 qui code pour le terminateur de la glucoamylase d'Aspergillus foetidus SE4.

Dans une variante préférée de l'invention, la séquence du promoteur de la glucoamylase et la séquence du terminateur de la glucoamylase proviennent du même champignon filamenteux. De bons résultats ont été obtenus avec un système d'expression, selon l'invention, comprenant la séquence du promoteur de la glucoamylase d'une souche d'Aspergillus foetidus et la séquence du terminateur de la glucoamylase d'une souche d'Aspergillus foetidus. D'excellents résultats ont été obtenus avec un système d'expression, selon l'invention, comprenant la séquence du promoteur de la glucoamylase de la souche d'Aspergillus foetidus SE4 et la séquence du terminateur de la glucoamylase de la souche d'Aspergillus foetidus SE4.

De préférence, dans le système d'expression selon l'invention, la séquence du promoteur est positionnée en amont de la séquence du signal de sécrétion, qui elle-même est positionnée en amont de la séquence mature; cette séquence mature est positionnée en amont de la séquence du terminateur. Ces positionnemments sont choisis de telle sorte que, dans des conditions appropriées, ils permettent l'expression de la glucose oxydase sous le contrôle des signaux de transcription, c'est-à-dire du promoteur et du terminateur.

De préférence, les séquences comprises dans le système d'expression sont liées de façon opérationnelle. La séquence du promoteur est liée de façon opérationnelle à la séquence du signal de sécrétion de la glucose oxydase. La séquence du signal de sécrétion de la glucose oxydase est liée de façon opérationnelle à la séquence mature de la glucose oxydase. La séquence mature de la glucose oxydase est liée de façon opérationnelle à la séquence du terminateur.

L'invention concerne également un procédé de préparation d'un système d'expression comprenant au moins la séquence d'un promoteur, la séquence du signal de sécrétion de la glucose oxydase, la séquence mature de la glucose oxydase, et la séquence d'un terminateur. Ce procédé comprend :
- l'isolement de la séquence du signal de sécrétion de la glucose oxydase et de la séquence mature de la glucose oxydase à partir de l'ADN génomique d'un microorganisme qui produit cette glucose oxydase, et
- l'introduction de la séquence du signal de sécrétion de la glucose oxydase et de la séquence mature de la glucose oxydase dans un vecteur contenant la séquence d'un promoteur, de préférence celui de la glucoamylase, et la séquence d'un terminateur, cette introduction étant faite dans un site choisi de telle sorte que le positionnement des séquences permette l'expression de la glucose oxydase sous le contrôle dudit promoteur et dudit terminateur.

L'invention concerne également un vecteur d'intégration contenant le système d'expression, tel que défini ci-dessus, et apte à permettre l'expression de la glucose oxydase.

Le principe de l'invention s'applique également à un vecteur d'expression contenant le système d'expression, tel que défini ci-dessus, et apte à permettre l'expression de la glucose oxydase.

Par vecteur d'intégration, on entend toute séquence d'ADN qui comprend une unité d'expression génique complète. Par unité d'expression génique complète, on entend le gène de structure et la ou les régions(s) du promoteur et la ou les région(s) de régulation nécessaires pour la transcription et la traduction. Par gène de structure, on entend la séquence codante qui est utilisée pour la transcription en ARN et permet la synthèse de la protéine par l'hôte.

De préférence, ce vecteur d'intégration est un plasmide. De bons résultats ont été obtenus avec le plasmide pFGOD.

L'invention concerne également une cellule transformée (cellule hôte) par le vecteur d'intégration défini ci-dessus. Cette cellule transformée est choisie de telle sorte que la séquence du promoteur, de préférence celui de la glucoamylase, et la séquence du terminateur comprises dans le système d'expression soient reconnues par cette cellule transformée, c'est-à-dire qu'elles sont compatibles et fonctionnelles pour cette cellule transformée, la séquence du promoteur, de préférence celui de la glucoamylase, et la séquence du terminateur comprises dans le système d'expression remplissent leurs fonctions respectives de signal de transcription, comme respectivement un promoteur et un terminateur, pour la cellule transformée.

Habituellement, la cellule transformée est une cellule de champignon filamenteux. Généralement, la cellule transformée est une cellule d'Aspergillus. De manière particulièrement préférée, la cellule transformée est une cellule d'Aspergillus niger, d'Aspergillus foetidus, d'Aspergillus awamori ou d'Aspergillus oryzae. De bons résultats ont été obtenus avec une cellule transformée d'Aspergillus niger, et plus particulièrement avec une cellule transformée de la souche d'Aspergillus niger NRRL 3. D'excellents résultats ont été obtenus avec une cellule transformée d'Aspergillus foetidus, et plus particulièrement avec une cellule transformée de la souche d'Aspergillus foetidus SE4.

Dans une variante préférée de l'invention, la cellule transformée par le vecteur d'intégration dérive de la souche d'où provient la séquence du promoteur et/ou la séquence du terminateur, séquence comprise dans le système d'expression. D'excellents résultats ont été obtenus avec une cellule transformée d'Aspergillus foetidus SE4. Cette cellule transformée par un vecteur d'intégration contient un système d'expression selon l'invention, ce système d'expression comprenant la séquence d'un promoteur, de préférence celui de la glucoamylase, d'Aspergillus foetidus SE4 et la séquence d'un terminateur, de préférence celui de la glucoamylase, d'Aspergillus foetidus SE4. Les meilleurs résultats ont été obtenus avec une cellule transformée par le vecteur d'intégration pFGOD.

Par champignon filamenteux, on entend les microorganismes eucaryotes qui comprennent toutes les formes filamenteuses de la division Eumycota. Dans cette division sont inclus les groupes Zygomycètes, Ascomycètes, Basidiomycètes et les champignons imparfaits incluant les Hyphomycètes. Les genres suivants sont compris dans cette définition : Aspergillus, Trichoderma, Neurospora, Podospora, Endothia, Mucor, Cochiobolus, Pyricularia, Penicillium, Humicola.

L'invention concerne également la souche purifiée et isolée d'Aspergillus foetidus SE4tr. Cette souche transformée SE4tr est obtenue à partir de la souche SE4. Elle diffère de cette souche SE4 par le fait qu'elle contient une ou plusieurs copies du plasmide pFGOD intégré dans son génome.

La souche d'Aspergillus foetidus SE4 a été obtenue à partir de la souche d'Aspergillus foetidus ATCC 14916 par mutagénèse et sélectionnée sur la base d'une production améliorée en glucoamylase.

La dénomination d'Aspergillus foetidus est synonyme d'Aspergillus citricus (ATCC Names of Industrial Fungi, 1994, p. 120, publié par The American Type Culture Collection).

L'invention concerne également un procédé pour la production extracellulaire de glucose oxydase caractérisé en ce qu'il comprend les étapes suivantes :
- transformation d'une cellule par un vecteur d'intégration contenant un système d'expression tel que défini ci-dessus dans des conditions permettant l'expression et la sécrétion de la glucose oxydase,
- culture de la cellule transformée dans un milieu de culture adéquat, et
- récupération de la glucose oxydase sécrétée.

Le milieu de fermentation obtenu après la culture de la cellule transformée contient la majeure partie de la glucose oxydase. En effet, la glucose oxydase est substantiellement sécrétée dans le milieu de culture par la souche transformée. La glucose oxydase obtenue par le procédé de l'invention est extracellulaire.

Le milieu de fermentation obtenu après la culture de la cellule transformée ne contient pratiquement pas de catalase. Ceci permet d'obtenir une composition enzymatique contenant la glucose oxydase substantiellement sans catalase.

L'invention concerne également la glucose oxydase produite par une cellule transformée telle que définie ci-dessus.

La glucose oxydase a de nombreuses applications industrielles, telles que, par exemple, dans les industries alimentaires, les industries pharmaceutiques et les industries chimiques.

Elle est notamment employée pour mesurer le taux de glucose dans le sang et peut, pour cette application, être incorporée dans un kit de diagnostic. La glucose oxydase obtenue selon l'invention présente un avantage dans cette application puisqu'elle n'est pas contaminée par de la catalase.

La glucose oxydase peut être utilisée pour mesurer la concentration de glucose et d'oxygène.

La glucose oxydase peut être utilisée comme anti-oxydant, pour enlever l'oxygène ou le glucose, notamment dans certains produits alimentaires ou certaines boissons.

La glucose oxydase peut également être introduite dans les dentifrices pour la prévention des caries.

La glucose oxydase a été incorporée à certaines peintures pour prévenir la corrosion.

La figure 1 représente la carte de restriction du plasmide pUCGOD.

La figure 2 représente la carte de restriction du plasmide pFGA1.

La figure 3 représente la carte de restriction du plasmide pFGA2.

La figure 4 représente la carte de restriction du plasmide pFGA2MUT.

La figure 5 représente la carte de restriction du plasmide pFGOD.

La figure 6 (figure 6a et figure 6b) représente la séquence de nucléotides (SEQ ID NO:7) codant pour le promoteur de la glucoamylase d'Aspergillus foetidus SE4.

La figure 7 représente la séquence de nucléotides (SEQ ID NO:8) codant pour le terminateur de la glucoamylase d'Aspergillus foetidus SE4.

Les symboles et abréviations utilisés dans ces figures sont explicités dans le tableau 1.

**TABLEAU 1**

| Symbole Abréviation | Signification |
|---|---|
| AMPR | gène apportant la résistance à l'ampicilline |
| ORI | origine de réplication dans E. coli |
| GOD | séquence codante de la glucose oxydase d'Aspergillus foetidus ATCC 14916 |
| 5' GA | promoteur de la glucoamylase d'Aspergillus foetidus SE4 |
| 3' GA | terminateur de la glucoamylase d'Aspergillus foetidus SE4 |
| GA | séquence codante de la glucoamylase d'Aspergillus foetidus SE4 |

La présente invention est illustrée par les exemples suivants.

### Exemple 1

### Isolement de la séquence codante de la glucose oxydase d'Aspergillus foetidus ATCC 14916

On réalise une culture de la souche d'Aspergillus foetidus ATCC 14916 sur un milieu nutritif liquide ACM ("Aspergillus Complete Medium") qui contient 2 % (poids/volume) d'extrait de malt, 0,1 % (poids/volume) de peptone (DIFCO) et 2 % (poids/volume) de glucose. Après une incubation de 48 heures à 32 °C, le mycélium est récolté.

L'ADN génomique de la souche d'Aspergillus foetidus ATCC 14916 est isolé selon la technique décrite dans GARBER, R.C. and YODER, O.L., Anal. Biochem. 135 (1983), pages 416-422.

On réalise le mélange suivant :

| | |
|---|---|
| H₂O distillée | 62 µl |
| Tampon PCR (10x) | 10 µl |
| dNTPs (2,5 mM chacun) | 8 µl |
| Oligonucléotide SEQ ID NO:1 (20 µM) | 5 µl |
| Oligonucléotide SEQ ID NO:2 (20 µM) | 5 µl |
| ADN génomique (dilutions 10⁻¹ à 10⁻⁴) | 10 µl |
| Taq-polymérase (5 U/µl) | 0,2 µl |

Le tampon PCR, sous la citation "(10x) Reaction Buffer composition", l'abréviation dNTPs, qui signifie tous les nucléotides dATP, dTTP, dGTP et dCTP, et le produit Taq-polymérase sont décrits dans la notice du kit vendu sous le nom "GENEAMP DNA AMPLIFICATION REAGENT KIT with AMPLITAQ Recombinant Taq DNA Polymerase" (PERKIN ELMER CETUS).

L'ADN génomique isolé de la souche d'Aspergillus foetidus ATCC 14916, est mis en oeuvre dans ce mélange à une concentration de 1 µg/µl.

La séquence codante de la glucose oxydase d'Aspergillus foetidus ATCC 14916 a été isolée selon la technique de la "PCR" (technique de la "Polymerase Chain Reaction" décrite dans SAIKI, R.K., et al., SCIENCE, 239 (1988), pages 487-491).

Les séquences des oligonucléotides synthétiques mis en oeuvre lors de cette étude sont les suivantes :
SEQ ID NO:1 
5′ - TGATGATCAGGATCCGGCGGCCGCACCTCAGCAATGCAGACTCTCCTTGTGAGCTCGCTTGTG - 3′
BamHI NotI
SEQ ID NO:2
5' - TCGATGAAGCTTCACTCACTGCATGGAAGCATAATCTTCCAAGATAGCATC - 3'
SEQ ID NO:3 
5′ - GATGCTATCTTGGAAGATTATGCTTCCATGCAGTGAGTGAAGCTTCATCGA - 3′
HindIII
La séquence SEQ ID NO:2 est le complément de la séquence SEQ ID NO:3.

La séquence de l'oligonucléotide SEQ ID NO:1 contient les informations pour les sites de restriction BamHI et NotI, les informations pour la région 5' non-traduite de la glucoamylase d'Aspergillus foetidus SE4 et le début de la séquence codante de la glucose oxydase.

La séquence de l'oligonucléotide SEQ ID NO:2 et, son complément, l'oligonucléotide SEQ ID NO:3 contiennent les nucléotides qui correspondent à la fin de la séquence codante de la glucose oxydase et les informations complémentaires pour le site de restriction HindIII.

La technique utilisée pour construire les oligonucléotides synthétiques est décrite dans BEAUCAGE, S.L. et al (1981), Tetrahedron Letters, 22, pages 1859-1882, en utilisant des β-cyanoéthyl phosphoramidites dans un appareil de BIOSEARCH CYCLONE SYNTHESIZER.

Les conditions suivies pour la "PCR" sont les suivantes : 2 minutes à 95 °C, puis 30 cycles d'une série comprenant 1 minute à 95 °C, 1 minute à 60 °C et 2 minutes à 72 °C, puis 10 minutes à 72 °C, puis une température de 20 °C est maintenue. Tous les autres paramètres utilisés pour cette "PCR" correspondent à ceux décrits dans la notice du kit vendu sous le nom "GENEAMP DNA AMPLIFICATION REAGENT KIT with AMPLITAQ Recombinant Taq DNA Polymerase" (PERKIN ELMER CETUS).

Un fragment d'ADN d'environ 1,8 kb (1 kb = 1000 paires de bases) a été isolé comme décrit ci-dessus. Il a été comparé à l'aide de la technique de l'analyse par restriction (analyse décrite dans Molecular Cloning - a laboratory manual - MANIATIS et al., (Cold Spring Harbor Laboratory) 1982, pages 374-379) à la séquence publiée dans The Journal of Biological Chemistry, 1990, 265 (7), pages 3793-3802. Aucune différence essentielle n'a été trouvée.

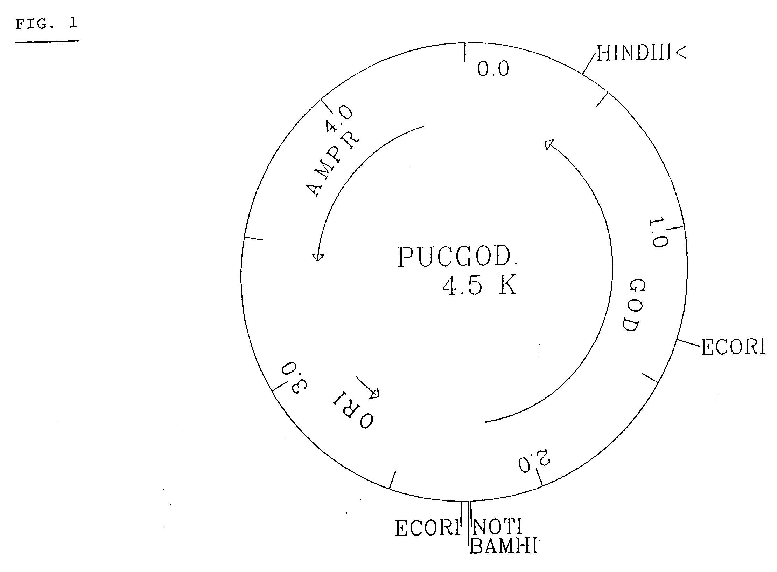

Le fragment d'ADN ainsi obtenu est digéré avec les enzymesde restriction BamHI et HindIII, puis est ligaturé, selon la technique de ligation décrite dans Molecular Cloning - a laboratory manual - SAMBROOK et al. - second edition, 1989, pages 1.68-1.69, avec le plasmide pUC18 (CLONTECH laboratories, n° 6110-1), qui a été préalablement digéré aux sites BamHI et HindIII. Le vecteur pUCGOD (figure 1) est ainsi obtenu.

### Exemple 2

### Construction du plasmide pFGA2MUT

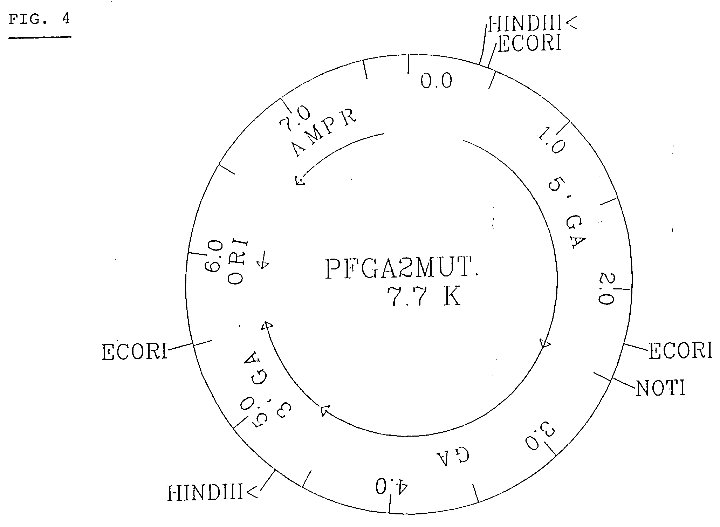

Le plasmide pFGA2MUT (figure 4) contient le gène de la glucoamylase d'Aspergillus foetidus SE4 dans lequel on a créé un site de clonage NotI entre le promoteur et la partie codante du gène de la glucoamylase, et un site de clonage HindIII entre la partie codante et le terminateur du gène de la glucoamylase. Ces deux sites de clonage NotI et HindIII sont créés par mutagénèse, comme décrit ci-après.

Le gène de la glucoamylase d'Aspergillus foetidus SE4 est isolé selon la description suivante.

L'ADN chromosomique de la souche d'Aspergillus foetidus SE4 est isolé selon la technique de GARBER et al., comme décrit à l'exemple 1. Cet ADN chromosomique isolé est digéré partiellement avec l'enzyme de restriction SauIIIA et les fragments ayant une taille de 8 à 10 kb sont isolés et purifiés par un gradient de sucrose (15 à 40 %) selon la technique décrite dans AUSUBEL, F.M. et al. (1989), Current Protocols in Molecular Biology (John WILLEY and Sons), pages 5.3.2-5.3.8. Ces fragments isolés et purifiés sont introduits dans le plasmide pBR322 (CLONTECH LABORATORIES catalogue n° 6210-1), qui a été préalablement digéré par l'enzyme de restriction BamHI. On transforme ensuite la souche d'E. coli DH5α (décrite par HANAHAN, D., J. Mol. Biol. (1983) 166, pages 557-580 et vendue par BETHESDA RESEARCH LABORATORIES (BRL), B.R.L. Focus (1986) 8, page 9).

Environ 15000 clones d'E. coli transformés sont criblés à l'aide de la technique d'hybridation (SAMBROOK et al., pages 9.52-9.55) en utilisant l'oligonucléotide synthétique suivant (SEQ ID NO:4) :
5' - GATTCATGGTTGAGCAACGAAGCGA - 3'
On se base sur la séquence de nucléotides de la glucoamylase publiée par BOEL et al., EMBO J. 3 (1984), pages 1097-1102.

On isole une colonie qui hybride avec cet oligonucléotide. On analyse les sites de restriction et on constate que cette souche contient le gêne complet de la glucoamylase, c'est-à-dire le promoteur, le terminateur et la région codante ainsi que le signal de sécrétion.

Le fragment de 8,7 kb a été introduit, comme décrit ci-dessus, dans le plasmide pBR322 au site BamHI.

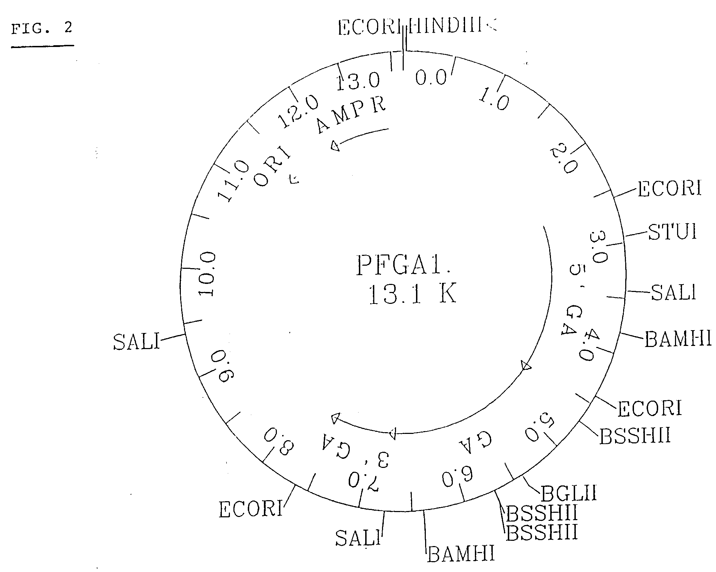

On crée ainsi le vecteur pFGA1 (figure 2), qui contient un insert d'ADN d'Aspergillus de 8,7 kb. Cet insert est la seule différence entre le plasmide pBR322 et le vecteur pFGA1.

On construit, comme suit, un plasmide qui est dérivé du vecteur pFGA1 et qui contient un insert moins grand que celui du vecteur pFGA1. Le vecteur pFGA1 est digéré par l'enzyme de restriction EcoRI, puis, à partir de ceci, on isole le fragment EcoRI d'une taille d'environ 5 kb contenant le promoteur, la région codante et le terminateur de la glucoamylase d'Aspergillus foetidus SE4 en suivant la méthode décrite par ZHU et al., 1985.

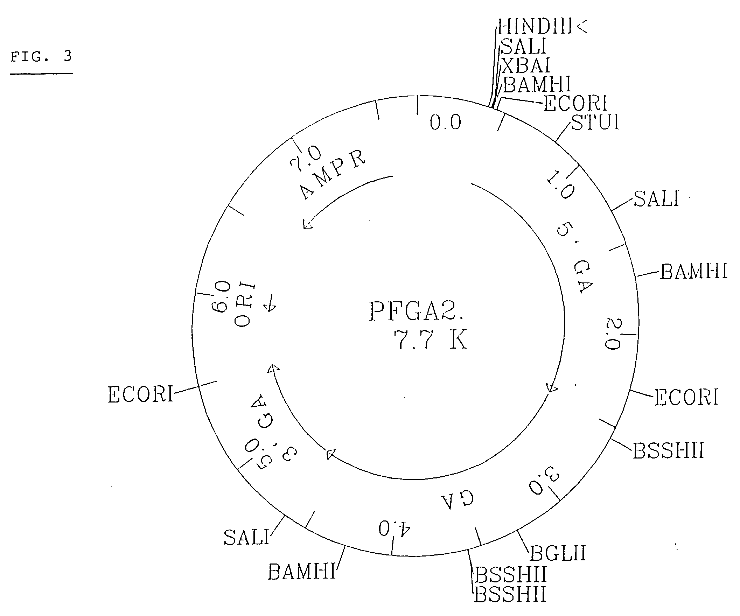

Ce fragment EcoRI est ensuite inséré dans le plasmide pUC18, qui a été préalablement digéré par l'enzyme de restriction EcoRI. On crée ainsi le vecteur pFGA2 (figure 3).

Les séquences nucléotidiques du promoteur et du terminateur de la glucoamylase d'Aspergillus foetidus SE4 ont été déterminées par la méthode de terminaison de chaînes utilisant des dideoxynucléotides, de SANGER et al. (1977) Proc. Natl. Acad. Sci. USA 74, pages 5463-5467.

L'analyse de cette séquence montre la présence d'un promoteur et d'un terminateur. On identifie la séquence de nucléotides (SEQ ID NO:7) codant pour le promoteur de la glucoamylase d'Aspergillus foetidus SE4. On identifie la séquence de nucléotides (SEQ ID NO:8) codant pour le terminateur de la glucoamylase d'Aspergillus foetidus SE4.

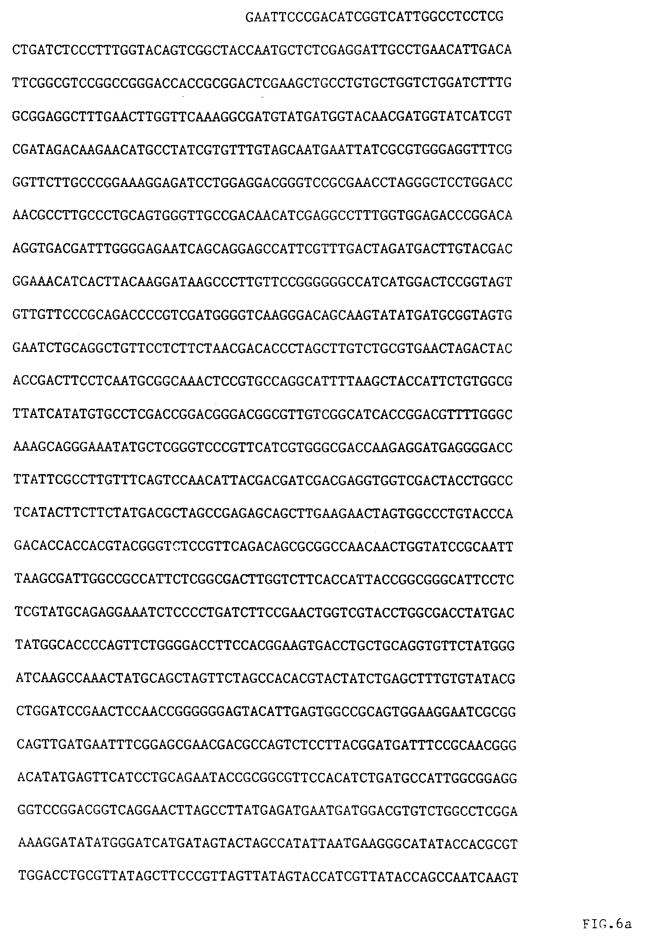

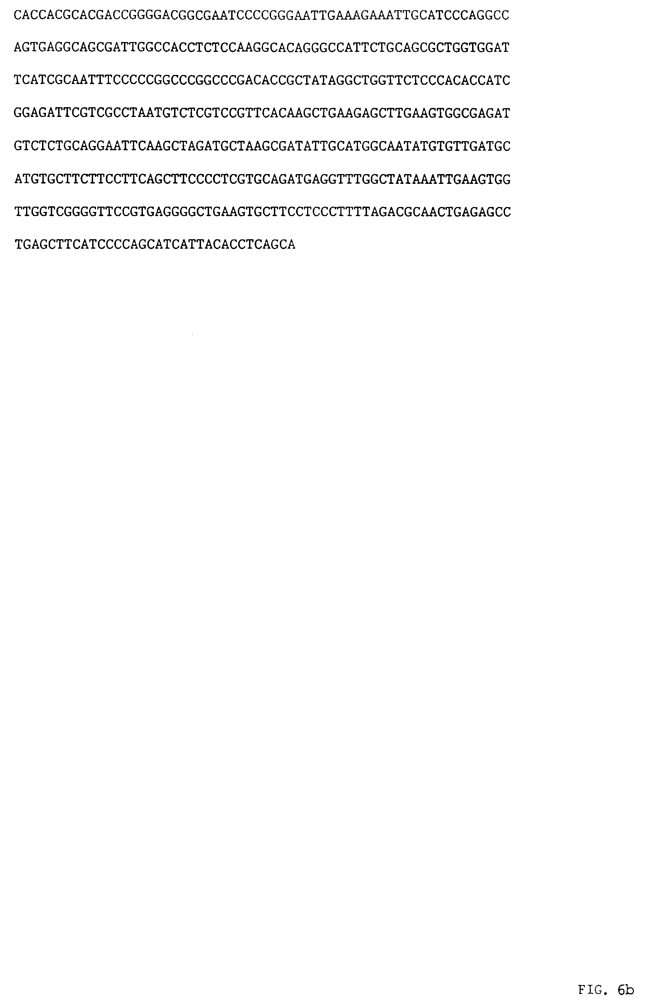

La figure 6 représente la séquence de nucléotides codant pour le promoteur de la glucoamylase d'Aspergillus foetidus SE4.

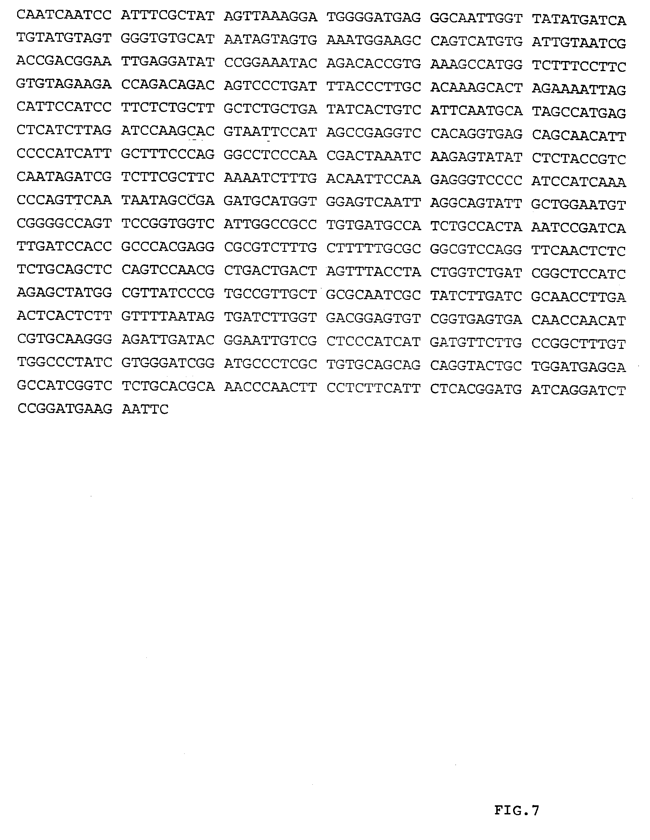

La figure 7 représente la séquence de nucléotides codant pour le terminateur de la glucoamylase d'Aspergillus foetidus SE4.

Les oligonucléotides synthétiques utilisés pour initier les réactions d'élongation par la T7 DNA polymérase ont été synthétisés par la méthode de BEAUCAGE et al. (1981) Tetrahedron letters 22, pages 1859-1882. Le séquençage a été effectué suivant le protocole donné par le fournisseur du kit de séquençage (PHARMACIA), en procédant à une dénaturation de l'ADN double brin par traitement avec du NaOH.

La stratégie de séquençage est décrite par SAMBROOK, 1989, pages 13.15 et 13.17. Les gels de polyacrylamide pour le séquençage ont été effectués suivant la technique décrite par SAMBROOK, 1989, pages 13.45-13.58.

On construit, comme suit, un plasmide qui est dérivé du plasmide pFGA2 et qui contient le gène de la glucoamylase d'Aspergillus foetidus SE4 dans lequel le promoteur et le terminateur sont séparés de la partie codante par des sites de restriction NotI et HindIII. On crée ainsi deux sites de restriction NotI et HindIII dans le plasmide pFGA2, qui contient le promoteur et le terminateur. Le promoteur et le terminateur de la glucoamylase d'Aspergillus foetidus SE4 sont séparés par des sites de restriction NotI et HindIII créés par deux mutagénèses dirigées suivant la procédure décrite dans la notice technique du kit de mutagénèse (BIORAD) "MUTA-GENE PHAGEMID in vitro Mutagenesis Kit".

Les oligonucléotides synthétiques utilisés au cours de cette procédure sont les suivants, respectivement SEQ ID NO:5 et SEQ ID NO:6 : 
5′ - GCCTGAGCTTCATCCCCAGCGCGGCCGCATCATTACACCTCAGCAATGT - 3′
NotI
5′ - TGACTGACACCTGGCGGTGAAAGCTTCAATCAATCCATTTCGCTATAGTT - 3′
HindIII
On obtient ainsi le vecteur pFGA2MUT qui contient le site de restriction NotI au début de la région 5' non-traduite du gène de la glucoamylase et le site de clivage HindIII après le stop-codon et dans la région 3' non-traduite de la glucoamylase.

### Exemple 3

### Construction du vecteur d'intégration

Suite à la digestion du plasmide pUCGOD, obtenu comme décrit à l'exemple 1, avec les deux enzymes de restriction NotI et HindIII, un fragment contenant la séquence codante de la glucose oxydase et la région 5' non-traduite du gêne de la glucoamylase d'Aspergillus foetidus SE4, est isolé en suivant la technique décrite par ZHU, J.D. et al., BIO/TECHNOLOGY, 3, 1985, pages 1014-1016.

La digestion partielle et compléte des plasmides avec des enzymes de restriction est effectuée en suivant la technique décrite par SAMBROOK et al. 1989, chapitres 5.28-5.32.

Ce fragment est introduit dans le site de clonage NotI-HindIII du plasmide pFGA2MUT, obtenu comme décrit à l'exemple 2. Ce plasmide contient le promoteur et le terminateur du gène de la glucoamylase d'Aspergillus foetidus SE4, séparés par les sites de clonage NotI et HindIII, créés par mutagènèse dirigée. La partie codante de la glucoamylase est donc remplacée par la partie codante de la glucose oxydase.

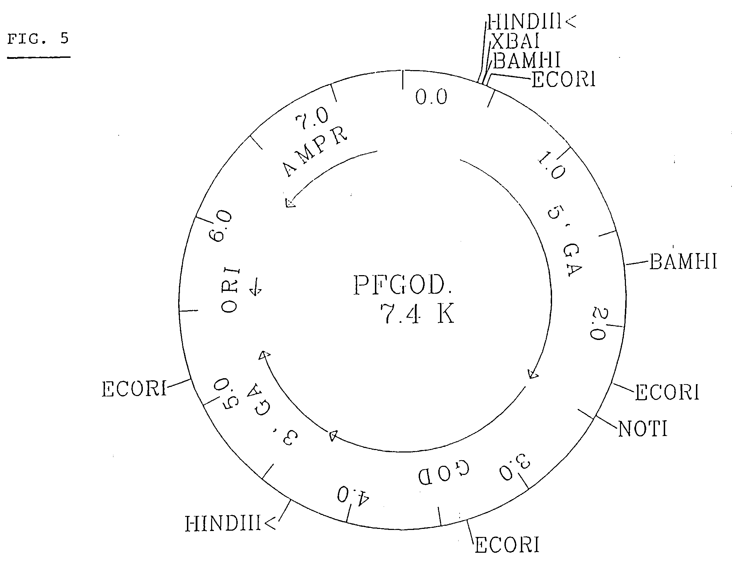

Le vecteur pFGOD (figure 5) est ainsi obtenu. Ce vecteur pFGOD contient la séquence codante de la glucose oxydase, qui comprend son propre signal de sécrétion, sous le contrôle des signaux de transcription de la glucoamylase d'Aspergillus foetidus. Ce vecteur contient le système d'expression qui comprend les séquences suivantes :
- la séquence du promoteur de la glucoamylase d'Aspergillus foetidus SE4,
- la séquence du signal de sécrétion de la glucose oxydase d'Aspergillus foetidus ATCC 14916,
- la séquence mature de la glucose oxydase d'Aspergillus foetidus ATCC 14916, et
- la séquence du terminateur de la glucoamylase d'Aspergillus foetidus SE4.

### Exemple 4

### Transformation de la souche d'Aspergillus foetidus SE4

La souche d'Aspergillus foetidus SE4 dérive de la souche d'Aspergillus foetidus ATCC 14916.

La souche d'Aspergillus foetidus ATCC 14916 est d'abord soumise à un traitement de mutagénèse par ultra-violet selon la technique décrite dans PONTECORVO, G., ROPER, J.A., HEMMONS, L.M., MACDONALD, K.D., and BUFTON, A.W.J., Advances in Genetics 5 (1953), pages 141-238.

Les souches traitées sont étalées sur un milieu nutritif gélosé POTATO DEXTROSE AGAR (DIFCO) auquel a été ajouté l'amidon (5 % en poids/volume). Après une incubation de 48 heures à 32 °C, les colonies présentant un halo d'hydrolyse d'amidon le plus grand sont prélevées et repiquées sur le milieu nutritif gélosé. On isole une souche produisant beaucoup de glucoamylase. Cette souche nommée SE4 présente des conidies brunes décolorées.

Le vecteur pFGOD est ensuite introduit dans la souche d'Aspergillus foetidus SE4 par cotransformation selon la technique décrite dans CARREZ et al., GENE, 94 (1990), pages 147-154.

Le plasmide p3SR2, qui contient le gène de l'acétamidase d'Aspergillus nidulans (amdS), est utilisé comme marqueur sélectif.

Le plasmide p3SR2 est décrit dans Hynes, M.J., Corrick, C.M., and King, J.A., Mol. Cell. Biol., 3 (1983), pages 1430-1439. On effectue la transformation avec ce plasmide selon la technique décrite par KELLY, J.M. and HYNES, M.J., EMBO J. 4 (1985), pages 475-479.

La souche d'Aspergillus foetidus SE4 a été transformée avec des quantités équimolaires des plasmides p3SR2 et pFGOD.

Environ 350 souches transformées sont obtenues. Ces souches transformées sont sélectionnées sur le milieu gélosé A contenant de l'ABTS (BOEHRINGER) pour détecter la production de glucose oxydase. Les colonies des souches transformées qui sécrètent aussi la glucose oxydase dans le milieu de culture, sont entourées d'un halo vert après 2 heures d'incubation à 32 °C.

Le milieu A est formé du milieu CZAPEK DOX AGAR (DIFCO) auquel on ajoute 10 % (poids/volume) de glucose, 0,05 % (poids/volume) d'ABTS (2,2'-azino-di-(3-éthylbenzthiazoline sulfonate)), (BOEHRINGER) et 8 mg/l de peroxydase de raifort (46 Purpurogallin Unités/mg, SIGMA).

Environ 40 % des souches transformées sécrètent la glucose oxydase, elles ont donc intégré, dans leur génome, le vecteur de sélection p3SR2 et le vecteur d'intégration pFGOD.

### Exemple 5

### Isolement et purification des souches transformées

Les souches transformées présentant le halo le plus grand sont isolées et repiquées sur un milieu gélosé POTATO DEXTROSE AGAR (DIFCO). 50 souches transformées sont mises en culture à 32 °C sur ce milieu jusqu'à sporulation.

Les spores sont récoltées, diluées dans de l'eau physiologique puis étalées sur le milieu gélosé A de telle sorte que des colonies individuelles soient obtenues. Les colonies venant d'une seule spore et présentant un halo large sont repiquées sur le milieu gélosé POTATO DEXTROSE AGAR. Ces colonies sont mises en culture à 32 °C sur ce milieu gélosé jusqu'à sporulation.

Les conidies purifiées des souches transformées sont récoltées et conservées. Ces souches transformées d'Aspergillus foetidus sont dénommées SE4tr.

### Exemple 6

### Production de glucose oxydase par la souche transformée d'Aspergillus foetidus SE4tr

Les conidies purifiées, obtenues à l'exemple 5, sont mises en culture dans un milieu liquide riche contenant de l'extrait de malt (DIFCO) (2 % en poids), de la peptone (DIFCO) (0,1 % en poids), de l'amidon hydrolysé (10 % en poids) et du carbonate de calcium (3,5 % en poids). Le pH du milieu de culture est ajusté à un pH 6 par l'addition d'ammoniac. La culture est réalisée à 32 °C sous agitation et est suivie durant 5 jours.

Puis, la culture obtenue est centrifugée à 5000 tours/minute pendant 15 minutes (BECKMAN JA-10). On mesure l'activité enzymatique (glucose oxydase) de la biomasse, après avoir cassé et disloqué les cellules par broyage de la biomasse gelée avec de l'azote liquide, (production dite intracellulaire) et du surnageant (production dite extracellulaire) selon la technique décrite dans FIEDUREK, J., et al., Enzyme Microb. Technol. 8 (1986), pages 734-736.

Les résultats d'activité enzymatique obtenus sont comparés avec ceux de la souche d'Aspergillus foetidus non transformée (souche SE4). La production intracellulaire de glucose oxydase de la souche transformée (souche SE4tr) en comparaison avec celle de la souche non transformée (souche SE4) est multipliée par un facteur de 5 à 10 et la production extracellulaire est multipliée par un facteur de 5000 à 10000.

On observe une forte augmentation de la production extracellulaire de glucose oxydase. En effet, la souche transformée sécrète presque la totalité de la glucose oxydase, qu'elle produit, dans le milieu de culture. La glucose oxydase produite par la souche non transformée n'est pratiquement pas sécrétée dans le milieu de culture. Une étape de dislocation des cellules de la souche non transformée est alors indispensable pour récupérer la glucose oxydase, ce qui n'est plus nécessaire avec la souche transformée selon l'invention. La production de glucose oxydase par la souche transformée SE4tr est substantiellement extracellulaire.

Par ailleurs, on ne détecte pratiquement pas de catalase dans le surnageant du milieu de fermentation obtenu après la culture de la souche transformée SE4tr.

### Exemple 7

### Profil d'activité en fonction du pH pour la glucose oxydase produite par la souche transformée SE4tr

On mesure l'activité enzymatique de la glucose oxydase à différents pH (de 3,5 à 9,0) à une température de 25 °C dans un tampon. Les conditions appliquées sont celles décrites à l'exemple 6.

On met en oeuvre le surnageant obtenu à l'exemple 6. Ce surnageant est dilué en fonction du pH et de l'activité enzymatique dans de l'eau distillée, puis dans du tampon citrate/phosphate 0,1 M pour la gamme de pH de 3,5 à 5,5 et dans du tampon KH₂PO₄/K₂HPO₄ 0,1 M pour la gamme de pH de 6,0 à 9,0.

On ajoute 2,5 ml d'une solution aqueuse de substrat à 50 microlitres de surnageant dilué à 25 °C. La-solution aqueuse de substrat contient 10 % (poids/volume) de glucose, 0,05 % (poids/volume) d'ABTS et 12 mg/l de peroxydase de raifort. Le pH de la solution aqueuse de substrat est ajusté avec du tampon citrate/phosphate 0,1 M pour la gamme de pH de 3,5 à 5,5 ou avec du tampon KH₂PO₄/K₂HPO₄ 0,1 M pour la gamme de pH de 6,0 à 9,0.

On mesure l'absorbance à 420 nm après une incubation de 2 minutes.

Les résultats sont rassemblés dans le tableau 2.

Au cours de cet essai l'activité enzymatique maximale a été mesurée pour l'échantillon placé à un pH d'environ 5,5 et à une température de 25 °C. Par définition on a donc attribué à cet échantillon une activité relative de 100 %.

Cet exemple montre que la glucose oxydase présente une activité enzymatique optimale mesurée à une température d'environ 25 °C dans une gamme de pH comprise entre environ 4,0 et environ 7,0.

**TABLEAU 2**

| pH | Activité relative en % | |
|---|---|---|
| | glucose oxydase produite par la souche | |
| | non transformée | transformée |
| 3,5 | 47 | 43 |
| 4,0 | 69 | 66 |
| 5,0 | 98 | 99 |
| 5,5 | 100 | 100 |
| 6,0 | 98 | 98 |
| 6,5 | 88 | 86 |
| 7,0 | 67 | 63 |
| 8,0 | 34 | 30 |
| 8,5 | 14 | 14 |
| 9,0 | 9 | 8 |

Le profil d'activité en fonction du pH de la glucose oxydase produite par la souche transformée est similaire à celui de la glucose oxydase produite par la souche non transformée, c'est à dire l'enzyme native.

### Exemple 8

### Profil d'activité en fonction de la température pour la glucose oxydase produite par la souche transformée SE4tr

On mesure l'activité enzymatique de la glucose oxydase à différentes températures (de 20 à 50 °C) à un pH de 6,0 dans le tampon KH₂PO₄/K₂HPO₄ 0,1 M. Les conditions appliquées sont celles décrites à l'exemple 6.

On met en oeuvre le surnageant tel qu'obtenu à l'exemple 6. Ce surnageant est dilué en fonction du pH et de l'activité enzymatique dans de l'eau distillée, puis dans du tampon KH₂PO₄/K₂HPO₄ 0,1 M.

On ajoute 2,5 ml d'une solution aqueuse de substrat à 50 microlitres de surnageant dilué. La solution aqueuse de substrat contient 10 % (poids/volume) de glucose, 0,05 % (poids/volume) d'ABTS et 12 mg/l de peroxydase de raifort. Le pH de la solution aqueuse de substrat est ajusté à un pH de 6,0 avec du tampon KH₂PO₄/K₂HPO₄ 0,1 M. Avant d'être mélangé, la solution aqueuse de substrat et le surnageant dilué sont chauffés à la température souhaitée.

On mesure l'absorbance à 420 nm après une incubation de deux minutes.

Les résultats sont rassemblés dans le tableau 3.

Au cours de cet essai l'activité enzymatique maximale a été mesurée pour l'échantillon placé à un pH d'environ 6,0 et à une température de 35 °C. Par définition on a donc attribué à cet échantillon une activité relative de 100 %.

Cet exemple montre que la glucose oxydase présente une activité enzymatique optimale mesurée à un pH d'environ 6,0 dans une gamme de température comprise entre environ 20 et environ 50 °C.

**TABLEAU 3**

| température °C | Activité relative en % | |
|---|---|---|
| | glucose oxydase produite par la souche | |
| | non transformée | transformée |
| 20 | 79 | 82 |
| 30 | 99 | 99 |
| 35 | 100 | 100 |
| 40 | 99 | 97 |
| 50 | 83 | 76 |

Le profil d'activité en fonction de la température de la glucose oxydase produite par la souche transformée est similaire à celui de la glucose oxydase produite par la souche non transformée, c'est-à-dire l'enzyme native.

Les exemples 7 et 8 montrent que les caractéristiques de la glucose oxydase produite par la souche transformée sont similaires à celles de la glucose oxydase produite par la souche non transformée.

## Revendications

1. Système d'expression utilisable pour la production extracellulaire de glucose oxydase, caractérisé en ce qu'il comprend :
- la séquence d'un promoteur,
- la séquence du signal de sécrétion de la glucose oxydase,
- la séquence mature de la glucose oxydase, et
- la séquence d'un terminateur.

2. Système d'expression selon la revendication 1, caractérisé en ce que la séquence du promoteur est celle du promoteur de la glucoamylase et la séquence du terminateur est celle du terminateur de la glucoamylase, ces deux séquences provenant de souches de champignons filamenteux.

3. Système d'expression selon la revendication 1 ou 2, caractérisé en ce que la séquence du signal de sécrétion de la glucose oxydase et la séquence mature de la glucose oxydase proviennent de souches de champignons filamenteux.

4. Système d'expression selon la revendication 2 ou 3, caractérisé en ce que la souche de champignon filamenteux est une souche d'Aspergillus.

5. Système d'expression selon la revendication 4, caractérisé en ce que la souche d'Aspergillus est une souche d'Aspergillus foetidus.

6. Système d'expression selon la revendication 1, caractérisé en ce que la séquence du promoteur de la glucoamylase et la séquence du terminateur de la glucoamylase proviennent de la souche d'Aspergillus foetidus SE4.

7. Système d'expression selon la revendication 1, caractérisé en ce que la séquence du signal de sécrétion de la glucose oxydase et la séquence mature de la glucose oxydase proviennent de la souche d'Aspergillus foetidus ATCC 14916.

8. Vecteur d'intégration contenant le système d'expression selon l'une quelconque des revendications précédentes, et apte à permettre l'expression de la glucose oxydase

9. Vecteur d'intégration selon la revendication 8, caractérisé en ce qu'il s'agit d'un plasmide.

10. Plasmide pFGOD.

11. Cellule transformée par le vecteur d'intégration selon la revendication 8 ou 9 ou par le plasmide selon la revendication 10.

12. Cellule transformée selon la revendication 11, caractérisée en ce qu'il s'agit d'une cellule de champignon filamenteux.

13. Cellule transformée selon la revendication 12, caractérisée en ce que la cellule de champignon filamenteux est une cellule d'Aspergillus.

14. Cellule transformée selon la revendication 13, caractérisée en ce que la cellule d'Aspergillus est une cellule d'Aspergillus foetidus.

15. Procédé pour la production extracellulaire de glucose oxydase caractérisé en ce qu'il comprend les étapes suivantes :
- transformation d'une cellule par un vecteur d'intégration contenant un système d'expression selon l'une quelconque des revendications 1 à 7 dans des conditions permettant l'expression et la sécrétion de la glucose oxydase,
- culture de la cellule transformée dans un milieu de culture adéquat, et
- récupération de la glucose oxydase sécrétée.

16. Souche isolée et purifiée d'Aspergillus foetidus SE4tr.

17. Promoteur de la glucoamylase d'Aspergillus foetidus.

18. Molécule d'ADN comprenant la séquence de nucléotides SEQ ID NO:7 qui code pour le promoteur de la glucoamylase d'Aspergillus foetidus SE4.

19. Terminateur de la glucoamylase d'Aspergillus foetidus.

20. Molécule d'ADN comprenant la séquence de nucléotides SEQ ID NO:8 qui code pour le terminateur de la glucoamylase d'Aspergillus foetidus SE4.

21. Glucose oxydase produite par une cellule transformée selon l'une quelconque des revendications 11, 12, 13 ou 14.
